# EUROPEAN PATENT APPLICATION

(11) **EP 2 119 789 A1**
(43) Date of publication of application: **18.11.2009**
(21) Application number: 08156332.2
(22) Date of filing: 16.05.2008
(51) Int. Cl.: C12P 1/04, C12N 15/09

(54) **Hyperproliferative recombinant cell**

(71) Applicant: Université Libre de Bruxelles, 1050 Bruxelles (BE)
(72) Inventor: Timmermans, Johan, B-1150 Bruxelles (BE); Van Melderen, Laurence, B-1410 Waterloo (BE)
(74) Representative: Van Malderen, Joëlle

(57) **Abstract**

The present invention is related to a method to increase the growth of a cell characterized by a deletion or one or more mutation(s) in the tldD and/or tldE genes, coupled with csrA partial deletion or deletion, to the obtained cell and to its use.

## Description

### Field of the invention

The present invention is related to a hyper-proliferative (recombinant) cell, its preparation process and its use, especially for the production of molecules of interest.

### Background of the invention and state of the art

Recombinant micro-organisms are used as such (in the form of a fertilizer, of a probiotic, of a vaccine, etc) or for the production of a variety of molecules, such as DNA constructs, polypeptides, etc in a cheap and flexible way.

However, a better yield of production requires culture of the micro-organisms, especially bacterial cells, at high densities and remains a difficulty.

Furthermore, fermentation is a consequence of micro-organism culture in broth, and results into large production of acetate, especially when the glycolysis overflows the Krebs cycle, inhibiting in turn their growth.

Several possibilities were implemented, such as dialysis, filtration and pure oxygen addition. The acetate separation from a micro-organism by dialysis or filtration allows the latter a further growth, with limitations, such as the price and a possible dilution of the obtained products in the medium. Oxygen addition favours oxidative metabolism instead of glycolysis, as well as acetate consumption. Furthermore, this method requires more sophisticated devices to be added to bioreactors.

The European Patent application EP0316229 describes a mutated *Escherichia coli* strain presenting reduced acetate production and increased cell density and subsequent yield. However, in this patent application, the obtained mutation(s), and the affected pathway(s) are not disclosed.

The group of Romeo (Wei, B., Shin, S., LaPorte, D., Wolfe, A. J. And Romeo, T. (2000) Global regulatory mutations in csrA and rpoS cause severe central carbon stress in Escherichia coli in the presence of acetate. J. Bacteriol. 182, 1632-1640) has identified a bacterial global regulator called csrA that activates glycolysis. However, the growth of the double csrA rpoS mutant is dramatically affected by acetate, while csrA mutant growth is delayed in acetate-containing medium. The expression of acetyl CoA synthetase, an enzyme required for oxidative metabolism, is down-regulated in CsrA mutants.

TldD and tldE genes were identified as necessary for microcin B17 processing and maturation (Allali, N., Afif, H., Couturier, M. and Van Melderen, L. (2002) The highly conserved TldD and TldE proteins of Escherichia coli are involved in microcin B17 processing and in CcdA degradation. J. Bacteriol, 184, 3224-3231). As the TldD and TldE proteins are highly conserved in many eu-and archae- bacteria, this suggests that they have other central roles.

Several methods were developed to ensure the correct insertion of a gene of interest. Patent application WO 02/066657 discloses *Escherichia coli* further having poison/anti-poison systems such as the protein CcdB and its antagonist CcdA, the protein Kid and its antagonist Kis, the protein Doc and its antagonist Phd, the protein HoK and its antagonist SoK, the RelE poison and its anti-poison RelB, PasA and its anti-poison PasB and PasC, MazE and its anti-poison MazF, and other poison molecules which are or are not of plasmid origin.

Other examples of toxic molecules for a procaryote cell are the protein encoded by the gene sacB (from *Bacillus amylolique-faciens*), the protein GpE, the protein GATA-1 or the protein Crp. The gene sacB encodes the levan sucrase which catalyzes the hydrolysis of sucrose into products which are toxic for *E*. *coli (*Pierce et al. (1992) Proc. Natl. Acad. Sci., 89, 2056-2060). The protein GpE causes lysis of E. coli cell and is encoded by the E gene from the bacteriophage ϕX174 which includes six unique restriction sites and (Heinrich et al. (1986) Gene, 42, 345-349). The protein GATA-1 has been described by Trudel et al. (Biotechniques 1996, 20, 684-693). The protein Crp has been described by Schlieper et al. (Anal. Biochem. 1998, 257, 203-209).

### Aims of the invention

The present invention aims to provide a new recombinant cell and its preparation process that do not present the drawbacks of the state of the art.

Preferably, the present invention aims to provide such cells that present improved properties, like a reduced acetate production and a reduced sensitivity to acetate, cells presenting an extended logarithmic growth phase and giving rise to a higher number of viable cells compared to the wild-type strain for improving (increasing) the production of valuable biological or chemical downstream products which mean endogenous and exogenous molecules obtained from these cells, like nucleic acid constructs, polypeptides, saccharides, lipids, vitamins, etc.

### Definitions

"A selection marker" is a gene that confers an advantage to a cell under selective pressure.

"A exogenous nucleotide sequence encoding for a gene product of interest" mean nucleotide sequences possibly involved in the production of biological or chemical compounds being these gene products of interest, that are not natively present in the (wild-type) cell and which presents an interest for industrial, medical, cosmetic, chemical or environmental applications. Nonlimiting examples of these genes products of interest are nucleotides sequences (possibly comprised in vectors, such as plasmids), viruses, phagemids, sacccharides (including oligo and polysaccharides), lipids (including fatty acids, such as omega-3 fatty acids), biopolymers, acids (including butyric acid), vitamins, hydrocarbons and derived products including polymers, hemes, enzymes and co-enzymes, bacteriocins, antibodies (or portions thereof, including nanobodies) nucleotide-based vaccination composition, (poly)peptides (or their portions such as epitopes) for vaccination and any protein that may be recovered for any industrial, medical, cosmetic, chemical, food-related or environmental application.

### Summary of the invention

A first aspect of the present invention is related to a method for improving (increasing) the growth of a cell, wherein said cell (wild-type cell) that comprises (natively) the tldD and/or tldE and/or csrA nucleotide sequences is submitted:
- to a partial or total deletion of the said tldD and/or tldE nucleotide sequence(s) or to one or more mutation(s) inactivating the said nucleotide sequences (i.e. inactivating the corresponding proteins) and
- to a partial deletion of the csrA nucleotide sequence or to one or more mutation(s) reducing the activity (partially inactivating) of the said CsrA protein.

These mutation(s) include mutation(s) in these tldD and/or tldE and/or csrA nucleotide sequence(s), preferably causing insertion or deletion of a nucleotide, and/or introducing a stop codon and/or resulting in a translated peptide having a substitution of one amino-acid by another, but also mutation(s) of nucleotide sequence(s) acting downstream and upstream these tldD and/or tlde and/or csrA sequences and resulting in the same phenotype.

According to the invention, the mutation inactivating the csrA sequence is preferably the csrA::kan mutation.

Preferably, the partially deleted csrA sequence is the csrA 1-50 sequence comprising the 5' region of the csrA open reading frame starting from nucleotide 1 to nucleotide 150.

Another aspect of the invention concerns an isolated (purified) hyperproliferative (recombinant) cell wherein the tldD and/or tldE nucleotide sequence(s) are partially or totally deleted or comprises one or more mutation(s) inactivating the said tldD and/or tldE nucleotide sequences and wherein the cell comprises a partially deleted csrA sequence preferably the csrA 1-50 sequence comprising the 5' region of the csrA open reading frame starting from nucleotide 1 to nucleotide 150, or one or more mutation(s) at least partially inactivating the CsrA protein, preferably the csrA::kan mutations and wherein the cell possibly further comprises an exogenous nucleotide sequence encoding a gene product of interest, with the exclusion that the cell is not E.coli.

These mutation(s) include mutation(s) in these tldD and/or tldE and/or csrA nucleotide sequence(s) for avoiding their expression, but also mutation(s) of nucleotide sequence(s) acting downstream and upstream these tldD and/or tldE and/or csrA sequences and resulting in the same phenotype.

Preferably, this cell is selected from the group consisting of Clostridium sp., Sphingomonas sp., Bacillus sp. and possibly the Lactobacillus sp., Bifidobacterium sp., Lactococcus sp. cells.

A further aspect of the present invention is related to an isolated and (purified) hyperproliferative (recombinant) E. coli cell wherein the tldD and/or tldE sequence are deleted or comprise one or more mutations inactivating the said tldD and/or tldE sequence, csrA sequence preferably the csrA 1-50 sequence comprising the 5' region of the csrA open reading frame starting from nucleotide 1 to nucleotide 150 or comprises one or more mutations inactivating the CsrA protein preferably the csrA::kan mutations and wherein the cell further comprises an exogenous nucleotide sequence encoding a gene product of interest.

These mutation(s) include mutation(s) in these tldD and/or tldE and/or csrA nucleotide sequence(s) for avoiding their expression, but also mutation(s) of nucleotide sequence(s) acting downstream and upstream these tldD and/or tldE and/or csrA sequences and resulting in the same phenotype.

In the method according to the invention the cell is preferably grown in a medium supplemented with a carbon source, preferably selected from the group consisting of glycerol, pyruvate or a glycolytic saccharide, being a (cheap) saccharide, such as glucose.

Furthermore, this recombinant cell may also further comprise one or more selective markers.

Advantageously, the selective marker is a poison or an anti-poison nucleotide sequence, preferably selected from the group consisting of CcdB/CcdA, Kid/Kis, Doc/Phd, Hok/Sok, RelE/RelB, PasA/PasB, PasA/PasC, MazE/MazF and other poisons/anti-poisons molecules which are or are not of plasmid origin (including active portions of these sequences possibly fused to coding or non coding sequences).

In another aspect of the present invention, the cell further includes (preferably in the chromosome of the cell) at least one nucleotide sequence encoding a poison protein (molecule) and includes (preferably on an extrachromosomal replicon) a nucleotide sequence encoding an inactive anti-poison protein (molecule) corresponding (antidote) to the said poison protein (molecule), which can be rendered active (functional) following correct integration of the nucleotide sequence encoding the gene product of interest in the cell (preferably in the extra-chromosomal replicon).

Advantageoulsy, the recombinant cell according to the invention could be used in any of the methods and kits described in the document WO 94/03616, WO 0146444, WO 02066657, WO 03078638 and WO 2004/022745. Therefore, this cell could be a prokaryote cell comprising in its genome a mutation providing resistance to activity of a poison protein (molecule), or may comprise in its genome (in its chromosome or on an extrachromosomal replicon) a nucleotide sequence encoding for anti-poison (antidote molecule) to the poison protein.

Preferably the mutation providing resistance to the activity of the poison protein being (molecule) CcdB is a mutation in the nucleotide sequence (gene) encoding A sub-unit or B sub-unit of gyrase and providing resistance to a poison protein, especially CcdB or active portion of this protein or fusion protein obtained from this wild type protein.

Advantageously, the cell according to the invention may further comprise in its genome one or more nucleotide sequence(s) conferring resistance to the toxic activity of a poison protein (molecule) used as selection marker. Preferably, this cell could corresponds to the bacterial cell having the deposit N° LMGP-1971 or the deposit N° LMGP-21399 that may further comprise also some specific nucleotide sequence(s) encoding a gene product(s) of interest and the above mentioned genetic modification(s) according to the present invention.

The cell according to the invention may further comprise possibly as selection marker(s) nucleotide sequence(s) encoding toxin protein(s) (molecule (s) being naturally or artificially toxic and affecting one or more vital functions of a (prokaryote) cell, such as the protein encoded by the gene sacB (from *Bacillus amylolique-faciens*), the protein GpE, the protein GATA 1 and the protein Crp. These toxic molecules or proteins are under the control of inducible promoter(s) and their corresponding nucleotide sequence(s) are rendered inactive after subsequent homologous recombination with a nucleotide cassette possibly bearing a nucleotide sequence encoding for a gene product of interest.

The inventors have also observed unexpectedly that the glycogen content in the recombinant cell of the invention is increased, by 10 fold, preferably by at least 50 fold compared to a control (wild type) cell which does not comprise these genetic modifications.

Furthermore, the inventors have observed that the recombinant cell of the invention presents also other phenotypes such as an impaired motility and a reduced acetate production. Preferably, in the recombinant cell of the invention, the acetate production is reduced by a factor of at least 10%, preferably of at least 15%, more preferably of at least 20%, compared to a production of a control (wild type) cell (a cell which does not present these genetic modifications).

The inventors have observed that the hyperproliferative cell of the invention presents an extended exponential growth which means that the growth is increased by at least 50%, preferably by at least 75%, more preferably by 100% compared to the growth of a control (wild type) cell that does not comprise these genetic modifications.

Advantageously, the lag period remains unaffected with the hyperproliferative cell according to the invention.

Surprisingly, the sensitivity of the hyperproliferative cell of the invention towards acetate is also reduced, preferably by a factor of at least 30%, more preferably of at least 50%, and advantageously of at least 75% compared to a control (wild type) cell that does not comprise these genetic modifications.

Therefore, another aspect of the present invention concerns method for obtaining a reduction of acetate production by a (recombinant) cell, preferably a reduction by a factor of at least 10%, preferably at least 15%, more preferably at least 20%, reduces the sensitivity of the cell towards acetate by a factor of at least 30%, more preferably at least 50%, advantageously by at least 75%.

The present invention is also related to a method to increase the glycogen content of a (recombinant) cell (increased by 10 fold, preferably by at least 50 fold) compared to a control (wild type) cell which comprises natively the tldD and/or tldE and csrA nucleotide sequence and wherein this cell is submitted to a partial or total deletion of the said tldD and/or tldE sequence(s) or to one or more mutation(s) inactivating the said nucleotide sequence (i.e. inactivating the corresponding protein) and to a partial deletion of the csrA nucleotide sequence or to one or more mutation(s) reducing the activity of the CsrA protein (partially inactivating the activity of the CsrA protein).

These mutation(s) include mutation(s) in the tldD and/or tldE and/or csrA nucleotide sequence for avoiding their expression, but also mutation(s) of nucleotide sequence(s) acting downstream and upstream this tldD and/or tldE and/or csrA sequence and resulting in the same phenotype.

Preferably, the mutation activating the csrA sequence is the csrA::kan mutation and the partially deleted csrA sequence is the csrA 1-50 sequence comprising the 5' region of the csrA open reading frame starting from nucleotide 1 to nucleotide 150.

Advantageously, the gene product of interest is an immunologically active gene product (immunosuppressive compound or immuno-stimulative compound) or may consist in a compound that is effective in degradation of an environmental pollutant, preferably a pesticidally active product.

The gene product of interest may also comprises or consist of polypeptides, such as enzymes, anti-thrombolitic compounds, hormones, neurotransmitters, antibodies, portion of antibodies, nanobodies, proteins involved in the synthesis of saccharides, vitamins, lipids, proteins involved in the transport of heavy metals, adjuvants to vaccine, etc.

Another aspect of the invention is the use of the hyperproliferative cell according to the invention for the synthesis of RNA and/or polypeptides (preferably insulin) or enzymes (including xyloglucanases, xylanases, lipases, esterases, etc), hormones, vitamins, hemes, lipids, amino-acids, acids such as butyric acid, saccharides (oligo and/or polysaccharides), biopolymers, hydrocarbons, carbonhydrates, antibiotic molecules, plasmids, viruses, phagemids, etc.

The hyperproliferative cell according to the invention may also be used as a probiotic or bioremediator.

### Short description of the figures

Figure 1 represents the growth of the MG1655 Escherichia coli strains and mutants in LB medium containing 0.4% (weight/volume) of glucose.

Figure 2 represents the growth of the MG1655 Escherichia coli strains and mutants in LB medium containing 0.4% (weight/volume) of arabinose.

### Detailed description of the invention

### Example 1: establishment and characterization of the strain according to the invention

Escherichia coli cells were engineered to have the deletion of tldD gene (SEQ.ID.N°1) and/or tldE gene (SEQ.ID.N°3) and/or the replacement of csrA sequence (SEQ.ID.N°5) by the truncated sequence csrA 1-50 (SEQ.ID.N°7). The sequence of truncated csrA 1-50 is: 
5′-
ATGCTGATTCTGACTCGTCGAGTTGGTGAGACCCTCATGATTGGGGATGAGGTCACCGT
GACAGTTTTAGGGGTAAAGGGCAACCAGGTACGTATTGGCGTAAATGCCCCGAAGGAAG
TTTCTGTTCACCGTGAAGAGATCTACCAGCGTTAA-3′
 Similar modification can be engineered in other prokaryotes or eukaryotes having the corresponding nucleotide sequences.

MG1655 (wild-type, laboratory K-12 strain) were engineered by removing the tldD and tldE Open Reading Frames (ORFs) (delta tldD mutant and delta tldE mutant, respectively) from the start codon to the last codon (leaving the stop codon) using the Datsenko and Wanner method (Datsenko and Wanner 2000). The csrA gene (SEQ.ID.N°5) was replaced by truncated sequence csrA 1-50 (SEQ.ID.N°7), with 
- 5'csrA1-50 primer (SEQ.ID.N°8)
CGAAGGAAGTTTCTGTTCACCGTGAAGAGATCTACCAGCGTTAAGTGTAGGCTGGAGCT
GCTTC
- 3'csrA1-50 primer (SEQ.ID.N°9)
   TTAGTAACTGGACTGCTGGGATTTTTCAGCCTGGATCATATGAATATCCTCCTTA

To delete a portion of csrA gene, the inventors used the Datsenko and Wanner method. They amplify by PCR an antibiotic resistance cassette using specific primers. At the 5' end of these primers, they added sequences homologous to the flanking regions of the insertion. The antibiotic resistance cassette sequence is inserted by homologous recombination. This cassette sequence is then removed by site-specific FLP-dependent recombination. The sequence homologous to the antibiotic resistance gene is underlined.

These three obtained single mutants have the same growth properties in LB and in minimal medium supplemented with glucose or other carbon sources than the corresponding wild-type cell strain.

The inventors tested if CsrA 1-50 truncated sequence could somehow have retained partial activity.

To test this hypothesis, the 5' region of the csrA ORF starting from nucleotide 1 to nucleotide 150 (1 nucleotide upstream of the insertion of the kan transposon) was cloned in the pKK223-3 vector (under the control of the inducible ptac promoter). A stop codon was added downstream of nucleotide 150.

Very surprisingly, the inventors found that (mutated) CsrA 1-50 keeps a residual activity.

The combination of the mutated csrA sequence (csrA::kan) sequence and either delta tldD or delta tldE mutants, hereafter referred to as "double mutants", results into a growth comparable to the wild type strain in LB medium but, very surprisingly, into a two-fold increase growth in glucose-enriched medium.

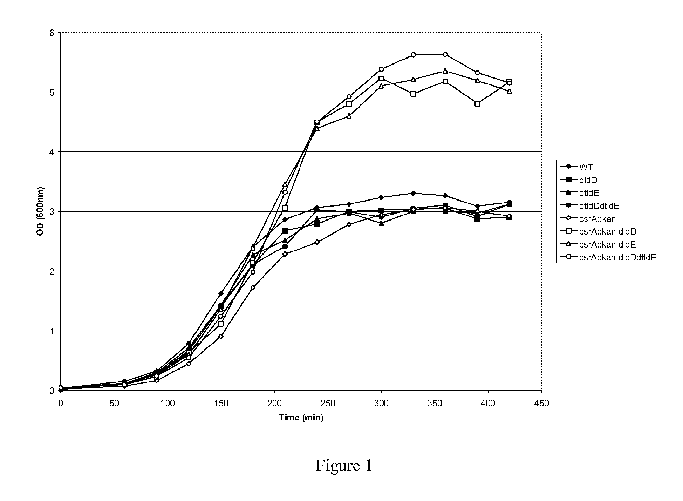

The characteristic of the mutant presenting a truncated csrA sequence and both tldD and tldE sequences (genes) deletion, hereafter referred to as "triple mutant" is presented in the Figure 1 (optical density at 600 nm, as function of time).

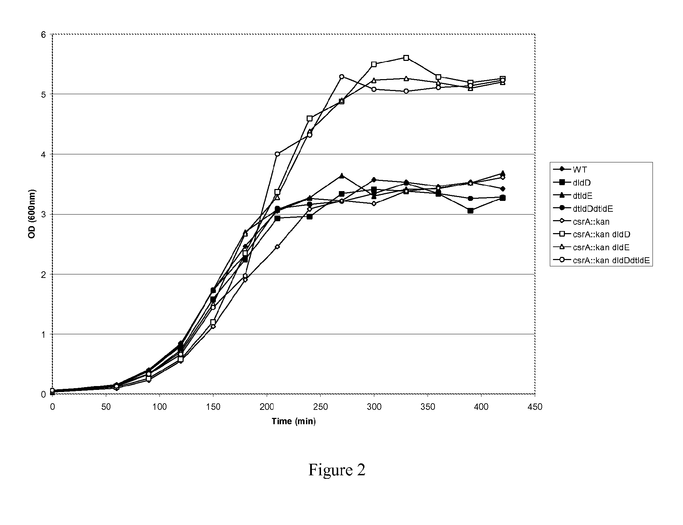

The addition of other carbon sources passing through glycolysis, such as arabinose (via fructose-6-phosphate), shows the same effects (Fig. 2). Advantageously, other carbon sources, such as glycerol and pyruvate provoke the same effect on increased growth.

In addition, glycogen storage of the mutant strain is advantageously increased by about 50-fold compared to a reference.

The synthesis of glycogen could be also used as a substrate for the productions of derived saccharides, such as maltodextrins that find some advantageous applications in the food industry.

Very surprisingly is the fact that for the same glucose consumption, these double and triple mutants of the invention produce less acetate, i.e. 0.6 g/l versus 0.9 g/l for wild-type strain.

Acetate production is measured using K-Acet kit (Megazyme). The skilled one may easily find other methods and adapt them to the present invention.

Also very surprising is the increased resistance (to 0.9 g/l) to acetate-inhibition of growth by the double and triple mutants in comparison to the wild-type strain (growth inhibited at 0.5 g/l).

Preferably, the hyper-proliferative cells (bacteria) of the present invention may further comprise selection markers, preferably genes involved in the resistance to antibiotics, or comprise the integration of one or more nucleotide sequences encoding toxins/antitoxins (or antidotes) also called poison/anti-poison sequences preferably selected from the group consisting of CcdB/CcdA, Kid/Kis, Doc/Phd, HoK/SoK, RelE/RelB, PasA/PasB and PasC, MazE/MazF, and other toxin/antidote molecules which are or are not of plasmid origin.

More preferably, the hyper-proliferative recombinant cells (bacteria) of the present invention have further stably incorporated through homologous recombination nucleotide sequences (placed under inducible promoters) encoding toxic proteins (molecules) from poison/anti-poison (antidote) of proteic killer gene systems that are kept or are further transformed with plasmid encoding anti-poison (antidote) to said toxic protein and a nucleotide sequence encoding a gene product of interest, before inducing the expression of poison nucleotide sequence to select or maintain confined clones of interest.

### Example 2: use of the modified cell strains according to the invention

According to the invention, recombinant cells (bacteria including *Escherichia coli* strains) were further developed incorporating an exogeneous (exogenous sequence not present natively in the cell) nucleotide sequence for expressing a protein of interest and advantageously the inventors measured its increased production.

The inventors have used recombinant bacteria according to the invention, preferably *Escherichia coli* strains, for the production of polypeptides, preferably insulin, and enzymes, including xyloglucanase, lipase and esterases.

The inventors have used recombinant bacteria according to the invention, preferably *Escherichia coli* strains, for the production of amino-acids, such as phenylalanine.

The inventors have used modified bacteria according to the invention for the production of peptidic and non-peptidic hormones.

Furthermore, the inventors have used recombinant bacteria according to the invention for the production of vitamins and coenzymes, such as menaquinones (vitamin K2), vitamin B12, coenzyme Q₁₀ and heme group.

The inventors have used modified bacteria according to the invention for the production of lipids, especially omega-3 fatty acids.

The inventors have used recombinant bacteria, such as *Clostridium* strains as described in WO2007/095215 for the production of acids such as butyric acid. Surprisingly, the inventors measured that the reduction of acetate synthesis of said recombinant bacteria, coupled to an increased growth, synergize to increase the yield of butyric acid. Advantageously, the inventors noticed that butyric acid may be converted into hexane after electrolysis.

The inventors have used recombinant bacteria according to the invention for the production of oligo and/or polysaccharides, such as xanthan gum, by *Xanthomonas campestris* and/or of other biopolymers.

The inventors have obtained recombinant *Sphingomonas* strains for an increased production of sphingan and/or of *Rhizobium meliloti strains* for the synthesis of succinoglycan polysaccharides.

The inventors have obtained recombinant *Escherichia coli* strains with nucleotide sequences described in WO2008/021141 for the increased production of ethanol from lignocellulosic biomass.

The inventors have obtained *Escherichia coli* strains according to the invention and further transformed with nucleotide sequences described in WO2008/003078 for the increased production of isoprenoid lipids. They adapt said methods to other prokaryotes, including photosynthetic species.

The inventors have modified *Escherichia coli* strains according to the invention and further with nucleotide sequences as described in Klocke et al. (Heterologous expression of enterocin A, a bacteriocin from *Enterococcus faecium*, fused to a cellulose-binding domain in *Escherichia coli* results in a functional protein with inhibitory activity against *Listeria*. (2005) Applied Genetics and Molecular Biotechnology, 67, 532-538.) for the production of bacteriocins (enterocin A and B).

The inventors have modified *Escherichia coli* strains according to the invention for an increased production of plasmids or fragments thereof that may comprise recombinant nucleotide sequences.

The person skilled in the art may also modify bacteria strains that are used as probiotics (live micro-organisms which, when administrated in adequate amounts, confer a health benefit on the host, especially in mammals including humans) or bio-remediators.

The person skilled in the art may use the modified bacteria according to the invention for an increased production of adjuvants to vaccines by further isolating DNA fragment sequences bearing immunostimulatory properties (such as CpG rich sequences) and possibly saccharides derivatives.

Beside an increased growth useful in the industrial processes, the inventors noticed an increased growth of the probiotic micro-organism in vivo.

The skilled one may easily adapt the present invention to other micro-organisms and to other industrial applications.

## Claims

1. A method for increasing the growth of a cell, wherein said cell comprising tldD and/or tldE and csrA sequences is submitted
- to a partial or total deletion of the said tldD and/or tldE nucleotide sequence(s) or to one or more mutation(s) inactivating the said tldD and/or tldE nucleotide sequences and
- to a partial deletion of the csrA nucleotide sequence or to one or more mutation(s) reducing the activity of the said CsrA protein sequence and preferably inactivating the said CsrA protein sequence.

2. **The** method according to claim 1, wherein the mutation reducing the activity, the csrA sequence is the csrA**::**kan mutation.

3. The method according to the claim 1, wherein the partially deleted csrA sequence is the csrA 1-50 sequence comprising the 5' region of the csrA open reading frame starting from nucleotide 1 to nucleotide 150.

4. The method of claims 1 to 3 wherein the cell is grown in a medium supplemented with carbon source, preferably glycerol or pyruvate and/or a glycolytic saccharide more preferably glucose.

5. The method according to any of the preceding claims, wherein the cell is a bacterial cell, preferably selected from the group consisting of *Escherichia coli, Clostridium* sp. and *Sphingomonas* sp.

6. The method according to any of the preceding claims, wherein the acetate production by the cell is reduced by a factor of at least 10%, preferably by 30%.

7. The method according to any of the preceding claims wherein the glycogen content in the cell is increased by at least 10 fold, preferably by at least 50 fold.

8. A recombinant cell wherein the tldD and/or tldE nucleotide sequence(s) are partially or totally deleted or comprises one or more mutation(s) inactivating the said tldD and/or tldE nucleotide sequences and wherein the cell comprises a partially deleted csrA sequence preferably the csrA 1-50 sequence comprising the 5' region of the csrA open reading frame starting from nucleotide 1 to nulcleotide 150 or one or more mutation(s) at least partially inactivating the CsrA protein preferably the csrA**::**kan mutations and wherein the cell possibly further comprises an exogenous nucleotide sequence encoding a gene product of interest, with the exclusion that the cell is not E.coli.

9. The cell of claim 8 which is selected from the group consisting of *Lactobacillus* sp., *Bifidobacterium* sp., *Clostridium* sp., *Sphingomonas* sp. and *Lactococcus lactis*.

10. A recombinant E.coli cell wherein the tldD and/or tldE sequences are deleted or comprises one or more mutations inactivating the said tldD and/or tldE sequences, wherein the cell comprises a partially deleted csrA sequence preferably the csrA 1-50 sequence comprising the 5' region of the csrA open reading frame starting from nucleotide 1 to nulcleotide 150 or comprises one or more mutation(s) inactivating the CsrA protein preferably the csrA**::**kan mutations and wherein the cell further comprises an exogenous nucleotide sequence encoding a gene product of interest.

11. The cell of claims 8 to 10, further comprising one or more selective markers, preferably a poison or an anti-poison nucleotide sequence, selected from the group consisting of CcdB/CcdA, Kid/Kis, Doc/phd, Hok/sok, relE/relB, PasA/PasB, PasA/PasC, MazE/MazF and others poison/anti-poison molecules which are or are not of plasmid origin.

12. The cell according to claims 8 to 11 further including (preferably in the chromosome of the cell) one or more nucleotide sequence(s) encoding a poison protein and (preferably upon an extra-chromosomal replicon) a nucleotide sequence encoding a corresponding inactive anti-poison protein antidote to the said poison, which can be rendered active following correct integration of the nucleotide sequence encoding the gene product of interest.

13. The use of the cell according to claims 8 to 12 for the production of endogenous or exogenous compounds.

14. The endogenous or exogenous compounds of claim 13 are selected from the group consisting of polypeptides, amino-acids, hormones, saccharides, biopolymers, vitamins, coenzymes, enzymes, hemes, lipids, acids, carbonhydrates, adjuvants to vaccine, bacteriocins, antibiotics, antibodies, plasmids, viruses, phagemids, RNA, DNA constructs or a mixture thereof.

15. The use of the cell according to claims 8 to 12 as a probiotic or bio-remediator.


SEQUENCE LISTING

<110>  ULB

<120>  HYPERPROLIFERATIVE RECOMBINANT CELL

<130>  ULBB 143

<160>  10

<170>  PatentIn version 3.3

<210>  1
<211>  1446
<212>  DNA
<213>  Escherichia coli

<220>
<221>  CDS
<222>  (1)..(1446)
<223>  tldD

<400>  1
atg agt ctt aac ctg gta agt gaa caa ttg cta gcg gcg aac ggc ctg      48
Met Ser Leu Asn Leu Val Ser Glu Gln Leu Leu Ala Ala Asn Gly Leu
1               5                   10                  15

aaa cat cag gac ttg ttc gcg atc ctc ggt caa ctg gcc gaa cgt cgc      96
Lys His Gln Asp Leu Phe Ala Ile Leu Gly Gln Leu Ala Glu Arg Arg
            20                  25                  30

ctt gat tat ggc gat ctc tat ttt cag tcg agc tat cac gaa tcc tgg      144
Leu Asp Tyr Gly Asp Leu Tyr Phe Gln Ser Ser Tyr His Glu Ser Trp
        35                  40                  45

gtt tta gaa gac cgc att att aaa gat ggt tct tac aac atc gat cag      192
Val Leu Glu Asp Arg Ile Ile Lys Asp Gly Ser Tyr Asn Ile Asp Gln
    50                  55                  60

ggc gtt ggt gtg cgt gca atc agc ggt gaa aaa acc gga ttt gct tac      240
Gly Val Gly Val Arg Ala Ile Ser Gly Glu Lys Thr Gly Phe Ala Tyr
65                  70                  75                  80

gct gac caa atc agc ctg ctg gcg ctg gaa cag agt gcg caa gcg gcg      288
Ala Asp Gln Ile Ser Leu Leu Ala Leu Glu Gln Ser Ala Gln Ala Ala
                85                  90                  95

cgc acc atc gtc cgt gat agt ggt gat ggt aaa gta cag acg ctg ggc      336
Arg Thr Ile Val Arg Asp Ser Gly Asp Gly Lys Val Gln Thr Leu Gly
            100                 105                 110

gcg gta gag cat agc ccg ttg tat acc tcg gta gat ccg ctg caa agc      384
Ala Val Glu His Ser Pro Leu Tyr Thr Ser Val Asp Pro Leu Gln Ser
        115                 120                 125

atg agc cgt gaa gag aag ctg gat atc ctg cgt cgc gtc gat aag gtt      432
Met Ser Arg Glu Glu Lys Leu Asp Ile Leu Arg Arg Val Asp Lys Val
    130                 135                 140

gcc cgc gaa gcg gac aag cgc gta cag gaa gtg act gcc agc ctc agt      480
Ala Arg Glu Ala Asp Lys Arg Val Gln Glu Val Thr Ala Ser Leu Ser
145                 150                 155                 160

ggt gtc tat gaa tta att ttg gtt gcg gcc acc gac ggc acg cta gcg      528
Gly Val Tyr Glu Leu Ile Leu Val Ala Ala Thr Asp Gly Thr Leu Ala
                165                 170                 175

gcg gat gtc cgt ccg ctg gtg cgt ctt tcc gtg agc gtt ctc gtc gaa 576
Ala Asp Val Arg Pro Leu Val Arg Leu Ser Val Ser Val Leu Val Glu
            180                 185                 190

gaa gat ggc aaa cgc gaa cgc ggt gcc agt ggc ggc ggc ggt cgt ttt 624
Glu Asp Gly Lys Arg Glu Arg Gly Ala Ser Gly Gly Gly Gly Arg Phe
        195                 200                 205

ggt tat gaa ttc ttc ctt gcc gat ctc gac ggc gaa gtc cgt gcg gat 672
Gly Tyr Glu Phe Phe Leu Ala Asp Leu Asp Gly Glu Val Arg Ala Asp
    210                 215                 220

gca tgg gca aaa gaa gca gtg cgt atg gcg ctg gtc aat ctt tct gcc 720
Ala Trp Ala Lys Glu Ala Val Arg Met Ala Leu Val Asn Leu Ser Ala
225                 230                 235                 240

gtt gct gca cca gcg ggc acc atg ccg gta gta ctt ggc gca ggt tgg 768
Val Ala Ala Pro Ala Gly Thr Met Pro Val Val Leu Gly Ala Gly Trp
                245                 250                 255

ccg ggc gtg ctg ttg cat gaa gcg gtt ggt cac ggt ctg gaa ggc gac 816
Pro Gly Val Leu Leu His Glu Ala Val Gly His Gly Leu Glu Gly Asp
            260                 265                 270

ttc aac cgc cgt ggc act tca gta ttt agt gga cag gtc ggg gag ctg 864
Phe Asn Arg Arg Gly Thr Ser Val Phe Ser Gly Gln Val Gly Glu Leu
        275                 280                 285

gtg gct tca gaa ctg tgt acc gtg gtt gat gat ggc acg atg gtc gat 912
Val Ala Ser Glu Leu Cys Thr Val Val Asp Asp Gly Thr Met Val Asp
    290                 295                 300

cgc cga ggt tcg gtg gcg att gat gac gaa ggt acg cca ggc cag tac 960
Arg Arg Gly Ser Val Ala Ile Asp Asp Glu Gly Thr Pro Gly Gln Tyr
305                 310                 315                 320

aac gtg ctg att gag aac ggc att ctg aaa ggc tac atg cag gat aaa 1008
Asn Val Leu Ile Glu Asn Gly Ile Leu Lys Gly Tyr Met Gln Asp Lys
                325                 330                 335

ctc aac gcg cgt ttg atg ggg atg acg ccg act ggc aac ggt cgc cgt 1056
Leu Asn Ala Arg Leu Met Gly Met Thr Pro Thr Gly Asn Gly Arg Arg
            340                 345                 350

gaa tcc tac gcc cat ctg ccc atg ccg cgt atg acc aac acc tat atg 1104
Glu Ser Tyr Ala His Leu Pro Met Pro Arg Met Thr Asn Thr Tyr Met
        355                 360                 365

ctg ccg ggt aaa tcg acc ccg cag gaa att att gaa tcc gtt gag tac 1152
Leu Pro Gly Lys Ser Thr Pro Gln Glu Ile Ile Glu Ser Val Glu Tyr
    370                 375                 380

ggt atc tat gca ccg aac ttt ggt ggc ggt cag gtg gat atc acc tcc 1200
Gly Ile Tyr Ala Pro Asn Phe Gly Gly Gly Gln Val Asp Ile Thr Ser
385                 390                 395                 400

ggc aaa ttc gtt ttc tcc act tca gaa gca tat ctg att gaa aac ggt 1248
Gly Lys Phe Val Phe Ser Thr Ser Glu Ala Tyr Leu Ile Glu Asn Gly
                405                 410                 415

aaa gta acg aag ccg gtg aaa ggc gca acg ttg att ggt tcc ggt atc 1296
Lys Val Thr Lys Pro Val Lys Gly Ala Thr Leu Ile Gly Ser Gly Ile
            420                 425                 430

gaa acc atg cag cag att tcg atg gtt ggc aac gac ctg aaa ctg gat 1344
Glu Thr Met Gln Gln Ile Ser Met Val Gly Asn Asp Leu Lys Leu Asp
        435                 440                 445

aac ggc gtg ggt gtc tgc ggt aaa gaa ggg caa agt ttg ccg gtt ggc     1392
Asn Gly Val Gly Val Cys Gly Lys Glu Gly Gln Ser Leu Pro Val Gly
    450                 455                 460

gtg ggc cag cca acg ttg aaa gtc gat aac ctg act gtt ggc ggt act     1440
Val Gly Gln Pro Thr Leu Lys Val Asp Asn Leu Thr Val Gly Gly Thr
465                 470                 475                 480

gcg taa                                                             1446
Ala

<210>  2
<211>  481
<212>  PRT
<213>  Escherichia coli

<400>  2

Met Ser Leu Asn Leu Val Ser Glu Gln Leu Leu Ala Ala Asn Gly Leu
1               5                   10                  15

Lys His Gln Asp Leu Phe Ala Ile Leu Gly Gln Leu Ala Glu Arg Arg
            20                  25                  30

Leu Asp Tyr Gly Asp Leu Tyr Phe Gln Ser Ser Tyr His Glu Ser Trp
        35                  40                  45

Val Leu Glu Asp Arg Ile Ile Lys Asp Gly Ser Tyr Asn Ile Asp Gln
    50                  55                  60

Gly Val Gly Val Arg Ala Ile Ser Gly Glu Lys Thr Gly Phe Ala Tyr
65                  70                  75                  80

Ala Asp Gln Ile Ser Leu Leu Ala Leu Glu Gln Ser Ala Gln Ala Ala
                85                  90                  95

Arg Thr Ile Val Arg Asp Ser Gly Asp Gly Lys Val Gln Thr Leu Gly
            100                 105                 110

Ala Val Glu His Ser Pro Leu Tyr Thr Ser Val Asp Pro Leu Gln Ser
        115                 120                 125

Met Ser Arg Glu Glu Lys Leu Asp Ile Leu Arg Arg Val Asp Lys Val
    130                 135                 140

Ala Arg Glu Ala Asp Lys Arg Val Gln Glu Val Thr Ala Ser Leu Ser
145                 150                 155                 160

Gly Val Tyr Glu Leu Ile Leu Val Ala Ala Thr Asp Gly Thr Leu Ala
                165                 170                 175

Ala Asp Val Arg Pro Leu Val Arg Leu Ser Val Ser Val Leu Val Glu
            180                 185                 190

Glu Asp Gly Lys Arg Glu Arg Gly Ala Ser Gly Gly Gly Gly Arg Phe
        195                 200                 205

Gly Tyr Glu Phe Phe Leu Ala Asp Leu Asp Gly Glu Val Arg Ala Asp
    210                 215                 220

Ala Trp Ala Lys Glu Ala Val Arg Met Ala Leu Val Asn Leu Ser Ala
225                 230                 235                 240

Val Ala Ala Pro Ala Gly Thr Met Pro Val Val Leu Gly Ala Gly Trp
                245                 250                 255

Pro Gly Val Leu Leu His Glu Ala Val Gly His Gly Leu Glu Gly Asp
            260                 265                 270

Phe Asn Arg Arg Gly Thr Ser Val Phe Ser Gly Gln Val Gly Glu Leu
        275                 280                 285

Val Ala Ser Glu Leu Cys Thr Val Val Asp Asp Gly Thr Met Val Asp
    290                 295                 300

Arg Arg Gly Ser Val Ala Ile Asp Asp Glu Gly Thr Pro Gly Gln Tyr
305                 310                 315                 320

Asn Val Leu Ile Glu Asn Gly Ile Leu Lys Gly Tyr Met Gln Asp Lys
                325                 330                 335

Leu Asn Ala Arg Leu Met Gly Met Thr Pro Thr Gly Asn Gly Arg Arg
            340                 345                 350

Glu Ser Tyr Ala His Leu Pro Met Pro Arg Met Thr Asn Thr Tyr Met
        355                 360                 365

Leu Pro Gly Lys Ser Thr Pro Gln Glu Ile Ile Glu Ser Val Glu Tyr
    370                 375                 380

Gly Ile Tyr Ala Pro Asn Phe Gly Gly Gly Gln Val Asp Ile Thr Ser
385                 390                 395                 400

Gly Lys Phe Val Phe Ser Thr Ser Glu Ala Tyr Leu Ile Glu Asn Gly
                405                 410                 415

Lys Val Thr Lys Pro Val Lys Gly Ala Thr Leu Ile Gly Ser Gly Ile
            420                 425                 430

Glu Thr Met Gln Gln Ile Ser Met Val Gly Asn Asp Leu Lys Leu Asp
        435                 440                 445

Asn Gly Val Gly Val Cys Gly Lys Glu Gly Gln Ser Leu Pro Val Gly
    450                 455                 460

Val Gly Gln Pro Thr Leu Lys Val Asp Asn Leu Thr Val Gly Gly Thr
465                 470                 475                 480

Ala

<210>  3
<211>  1353
<212>  DNA
<213>  Escherichia coli

<220>
<221>  CDS
<222>  (1)..(1353)
<223>  tlDE

<400>  3
atg gca ctt gca atg aaa gta atc tct caa gtt gaa gcg cag cgc aag      48
Met Ala Leu Ala Met Lys Val Ile Ser Gln Val Glu Ala Gln Arg Lys
1               5                   10                  15

att ctg gaa gaa gca gtt tcc act gcg ctg gag ttg gcc tca ggc aaa      96
Ile Leu Glu Glu Ala Val Ser Thr Ala Leu Glu Leu Ala Ser Gly Lys
            20                  25                  30

tcg gac ggt gcg gaa gtt gcc gtc agc aag acc acc ggc att agc gta      144
Ser Asp Gly Ala Glu Val Ala Val Ser Lys Thr Thr Gly Ile Ser Val
        35                  40                  45

agc acg cgt tat ggt gaa gtg gag aat gtt gaa ttc aat agc gat ggc      192
Ser Thr Arg Tyr Gly Glu Val Glu Asn Val Glu Phe Asn Ser Asp Gly
    50                  55                  60

gcg ctg ggg atc act gtt tat cac cag aac cgc aaa ggt agc gca tca      240
Ala Leu Gly Ile Thr Val Tyr His Gln Asn Arg Lys Gly Ser Ala Ser
65                  70                  75                  80

tcc acc gat tta agc ccg cag gcc att gcc cgt act gta cag gcg gcg      288
Ser Thr Asp Leu Ser Pro Gln Ala Ile Ala Arg Thr Val Gln Ala Ala
                85                  90                  95

ctg gat att gcc cgt tat acc tcg cca gat ccc tgt gcc ggc gtg gca      336
Leu Asp Ile Ala Arg Tyr Thr Ser Pro Asp Pro Cys Ala Gly Val Ala
            100                 105                 110

gac aaa gag ctg ctg gcc ttt gac gca cca gat ctc gac ttg ttc cac      384
Asp Lys Glu Leu Leu Ala Phe Asp Ala Pro Asp Leu Asp Leu Phe His
        115                 120                 125

cct gcg gaa gtt tcc ccg gat gaa gcc att gaa ctg gcg gcc cgc gca      432
Pro Ala Glu Val Ser Pro Asp Glu Ala Ile Glu Leu Ala Ala Arg Ala
    130                 135                 140

gaa cag gcg gca ttg cag gcg gac aaa cgc atc acc aat acc gaa ggt      480
Glu Gln Ala Ala Leu Gln Ala Asp Lys Arg Ile Thr Asn Thr Glu Gly
145                 150                 155                 160

ggc agc ttt aac agc cac tac ggt gtc aaa gtt ttt ggc aac agc cac      528
Gly Ser Phe Asn Ser His Tyr Gly Val Lys Val Phe Gly Asn Ser His
                165                 170                 175

ggc atg ttg cag ggt tac tgc tca acg cgt cat tcg ctc tcc agc tgt      576
Gly Met Leu Gln Gly Tyr Cys Ser Thr Arg His Ser Leu Ser Ser Cys
            180                 185                 190

gta att gcc gaa gaa aat ggc gat atg gag cgt gat tac gcc tac acc 624
Val Ile Ala Glu Glu Asn Gly Asp Met Glu Arg Asp Tyr Ala Tyr Thr
        195                 200                 205

att ggt cgt gcg atg agc gat ctg caa acg cca gag tgg gtt ggg gcc 672
Ile Gly Arg Ala Met Ser Asp Leu Gln Thr Pro Glu Trp Val Gly Ala
    210                 215                 220

gac tgt gct cgc cgt act tta tcg cgt ctg tca ccg cgt aaa ctc tcc 720
Asp Cys Ala Arg Arg Thr Leu Ser Arg Leu Ser Pro Arg Lys Leu Ser
225                 230                 235                 240

acc atg aaa gcg cca gtc att ttt gcc aat gaa gtg gca acc ggg ctt 768
Thr Met Lys Ala Pro Val Ile Phe Ala Asn Glu Val Ala Thr Gly Leu
                245                 250                 255

ttt ggg cat ctg gtg ggg gcg ata gcg ggt gga tcg gtt tat cgt aaa 816
Phe Gly His Leu Val Gly Ala Ile Ala Gly Gly Ser Val Tyr Arg Lys
            260                 265                 270

tct acc ttc ctg ctg gat tcg ctg ggt aaa caa att ctg ccg gac tgg 864
Ser Thr Phe Leu Leu Asp Ser Leu Gly Lys Gln Ile Leu Pro Asp Trp
        275                 280                 285

ctg acc att gaa gag cat ccg cat ctg ctg aaa ggg ctg gcg tcg acg 912
Leu Thr Ile Glu Glu His Pro His Leu Leu Lys Gly Leu Ala Ser Thr
    290                 295                 300

cca ttc gac agc gaa ggt gtg cgc acc gag cgt cgc gat att att aaa 960
Pro Phe Asp Ser Glu Gly Val Arg Thr Glu Arg Arg Asp Ile Ile Lys
305                 310                 315                 320

gat ggc atc ctg act cag tgg ctg ctg acc agc tac tcg gcg cgg aaa 1008
Asp Gly Ile Leu Thr Gln Trp Leu Leu Thr Ser Tyr Ser Ala Arg Lys
                325                 330                 335

ctg ggg ctg aaa agc acc gga cat gcg ggc ggt att cac aac tgg cgg 1056
Leu Gly Leu Lys Ser Thr Gly His Ala Gly Gly Ile His Asn Trp Arg
            340                 345                 350

att gcc gga caa ggt cta agc ttc gag cag atg ctc aaa gag atg ggc 1104
Ile Ala Gly Gln Gly Leu Ser Phe Glu Gln Met Leu Lys Glu Met Gly
        355                 360                 365

acc ggg ctg gtg gtg acg gaa ttg atg ggc cag ggc gtg agt gcc att 1152
Thr Gly Leu Val Val Thr Glu Leu Met Gly Gln Gly Val Ser Ala Ile
    370                 375                 380

acc ggt gat tat tcc cgt ggt gca gcg ggc ttc tgg gta gag aat ggc 1200
Thr Gly Asp Tyr Ser Arg Gly Ala Ala Gly Phe Trp Val Glu Asn Gly
385                 390                 395                 400

gaa att cag tat ccg gtg agc gaa atc acc atc gca ggt aat tta aaa 1248
Glu Ile Gln Tyr Pro Val Ser Glu Ile Thr Ile Ala Gly Asn Leu Lys
                405                 410                 415

gat atg tgg cgc aat att gtc acc gtc ggt aac gat att gaa aca cgc 1296
Asp Met Trp Arg Asn Ile Val Thr Val Gly Asn Asp Ile Glu Thr Arg
            420                 425                 430

agt aat ata cag tgt ggt tct gtg ctg ttg ccg gag atg aaa atc gcc 1344
Ser Asn Ile Gln Cys Gly Ser Val Leu Leu Pro Glu Met Lys Ile Ala
        435                 440                 445

gga cag taa 1353
Gly Gln
    450

<210>  4
<211>  450
<212>  PRT
<213>  Escherichia coli

<400>  4

Met Ala Leu Ala Met Lys Val Ile Ser Gln Val Glu Ala Gln Arg Lys
1               5                   10                  15

Ile Leu Glu Glu Ala Val Ser Thr Ala Leu Glu Leu Ala Ser Gly Lys
            20                  25                  30

Ser Asp Gly Ala Glu Val Ala Val Ser Lys Thr Thr Gly Ile Ser Val
        35                  40                  45

Ser Thr Arg Tyr Gly Glu Val Glu Asn Val Glu Phe Asn Ser Asp Gly
    50                  55                  60

Ala Leu Gly Ile Thr Val Tyr His Gln Asn Arg Lys Gly Ser Ala Ser
65                  70                  75                  80

Ser Thr Asp Leu Ser Pro Gln Ala Ile Ala Arg Thr Val Gln Ala Ala
                85                  90                  95

Leu Asp Ile Ala Arg Tyr Thr Ser Pro Asp Pro Cys Ala Gly Val Ala
            100                 105                 110

Asp Lys Glu Leu Leu Ala Phe Asp Ala Pro Asp Leu Asp Leu Phe His
        115                 120                 125

Pro Ala Glu Val Ser Pro Asp Glu Ala Ile Glu Leu Ala Ala Arg Ala
    130                 135                 140

Glu Gln Ala Ala Leu Gln Ala Asp Lys Arg Ile Thr Asn Thr Glu Gly
145                 150                 155                 160

Gly Ser Phe Asn Ser His Tyr Gly Val Lys Val Phe Gly Asn Ser His
                165                 170                 175

Gly Met Leu Gln Gly Tyr Cys Ser Thr Arg His Ser Leu Ser Ser Cys
            180                 185                 190

Val Ile Ala Glu Glu Asn Gly Asp Met Glu Arg Asp Tyr Ala Tyr Thr
        195                 200                 205

Ile Gly Arg Ala Met Ser Asp Leu Gln Thr Pro Glu Trp Val Gly Ala
    210                 215                 220

Asp Cys Ala Arg Arg Thr Leu Ser Arg Leu Ser Pro Arg Lys Leu Ser
225                 230                 235                 240

Thr Met Lys Ala Pro Val Ile Phe Ala Asn Glu Val Ala Thr Gly Leu
                245                 250                 255

Phe Gly His Leu Val Gly Ala Ile Ala Gly Gly Ser Val Tyr Arg Lys
            260                 265                 270

Ser Thr Phe Leu Leu Asp Ser Leu Gly Lys Gln Ile Leu Pro Asp Trp
        275                 280                 285

Leu Thr Ile Glu Glu His Pro His Leu Leu Lys Gly Leu Ala Ser Thr
    290                 295                 300

Pro Phe Asp Ser Glu Gly Val Arg Thr Glu Arg Arg Asp Ile Ile Lys
305                 310                 315                 320

Asp Gly Ile Leu Thr Gln Trp Leu Leu Thr Ser Tyr Ser Ala Arg Lys
                325                 330                 335

Leu Gly Leu Lys Ser Thr Gly His Ala Gly Gly Ile His Asn Trp Arg
            340                 345                 350

Ile Ala Gly Gln Gly Leu Ser Phe Glu Gln Met Leu Lys Glu Met Gly
        355                 360                 365

Thr Gly Leu Val Val Thr Glu Leu Met Gly Gln Gly Val Ser Ala Ile
    370                 375                 380

Thr Gly Asp Tyr Ser Arg Gly Ala Ala Gly Phe Trp Val Glu Asn Gly
385                 390                 395                 400

Glu Ile Gln Tyr Pro Val Ser Glu Ile Thr Ile Ala Gly Asn Leu Lys
                405                 410                 415

Asp Met Trp Arg Asn Ile Val Thr Val Gly Asn Asp Ile Glu Thr Arg
            420                 425                 430

Ser Asn Ile Gln Cys Gly Ser Val Leu Leu Pro Glu Met Lys Ile Ala
        435                 440                 445

Gly Gln
    450


<210>  5
<211>  186
<212>  DNA
<213>  Escherichia coli


<220>
<221>  CDS
<222>  (1)..(186)
<223>  csrA

<400>  5

atg ctg att ctg act cgt cga gtt ggt gag acc ctc atg att ggg gat 48
Met Leu Ile Leu Thr Arg Arg Val Gly Glu Thr Leu Met Ile Gly Asp
1               5                   10                  15

gag gtc acc gtg aca gtt tta ggg gta aag ggc aac cag gta cgt att 96
Glu Val Thr Val Thr Val Leu Gly Val Lys Gly Asn Gln Val Arg Ile
            20                  25                  30

ggc gta aat gcc ccg aag gaa gtt tct gtt cac cgt gaa gag atc tac 144
Gly Val Asn Ala Pro Lys Glu Val Ser Val His Arg Glu Glu Ile Tyr
        35                  40                  45

cag cgt atc cag gct gaa aaa tcc cag cag tcc agt tac taa 186
Gln Arg Ile Gln Ala Glu Lys Ser Gln Gln Ser Ser Tyr
    50                  55                  60

<210> 6
<211> 61
<212> PRT
<213> Escherichia coli

<400> 6

Met Leu Ile Leu Thr Arg Arg Val Gly Glu Thr Leu Met Ile Gly Asp
1               5                   10                  15

Glu Val Thr Val Thr Val Leu Gly Val Lys Gly Asn Gln Val Arg Ile
            20                  25                  30

Gly Val Asn Ala Pro Lys Glu Val Ser Val His Arg Glu Glu Ile Tyr
        35                  40                  45

Gln Arg Ile Gln Ala Glu Lys Ser Gln Gln Ser Ser Tyr
    50                  55                  60

<210> 7
<211> 153
<212> DNA
<213> Escherichia coli

<220>
<221> CDS
<222> (1)..(153)
<223> csrA 1-50

<400> 7
atg ctg att ctg act cgt cga gtt ggt gag acc ctc atg att ggg gat 48
Met Leu Ile Leu Thr Arg Arg Val Gly Glu Thr Leu Met Ile Gly Asp
1               5                   10                  15

gag gtc acc gtg aca gtt tta ggg gta aag ggc aac cag gta cgt att 96
Glu Val Thr Val Thr Val Leu Gly Val Lys Gly Asn Gln Val Arg Ile
            20                  25                  30

ggc gta aat gcc ccg aag gaa gtt tct gtt cac cgt gaa gag atc tac 144
Gly Val Asn Ala Pro Lys Glu Val Ser Val His Arg Glu Glu Ile Tyr
        35                  40                  45

cag cgt taa 153
Gln Arg
    50

<210>  8
<211>  50
<212>  PRT
<213>  Escherichia coli

<400>  8

Met Leu Ile Leu Thr Arg Arg Val Gly Glu Thr Leu Met Ile Gly Asp
1               5                   10                  15

Glu Val Thr Val Thr Val Leu Gly Val Lys Gly Asn Gln Val Arg Ile
            20                  25                  30

Gly Val Asn Ala Pro Lys Glu Val Ser Val His Arg Glu Glu Ile Tyr
        35                  40                  45

Gln Arg
    50

<210>  9
<211>  64
<212>  DNA
<213>  Artificial Sequence

<220>
<223>  5' csrA primer

<400>  9
cgaaggaagt ttctgttcac cgtgaagaga tctaccagcg ttaagtgtag gctggagctg      60

cttc                                                                  64

<210>  10
<211>  55
<212>  DNA
<213>  Artificial sequence

<220>
<223>  3' csrA 1-50 primer

<400>  10
ttagtaactg gactgctggg atttttcagc ctggatcata tgaatatcct cctta          55